# EUROPÄISCHE PATENTSCHRIFT

(11) **EP 0 813 862 B1**
(45) Veröffentlichungstag und Bekanntmachung des Hinweises auf die Patenterteilung: **18.12.2002**
(21) Anmeldenummer: 97109291.1
(22) Anmeldetag: 09.06.1997
(51) Int. Cl.: A61K 7/46, A61K 7/00

(54) **Parfümölkonzentrate in Form transparenter wässriger Mikroemulsion**
Perfume oil concentrates in the form of transparent aqueous microemulsions
Concentrés de parfum sous forme de microémulsions aqueuses transparantes

(30) Priorität: 17.06.1996 DE 19624051
(43) Veröffentlichungstag der Anmeldung: 29.12.1997
(73) Patentinhaber: Cognis Deutschland GmbH & Co. KG, 40589 Düsseldorf (DE)
(72) Erfinder: Förster, Thomas, Dr.-Dipl.-Chem., 40699 Erkrath (DE); Claas, Marcus, 40723 Hilden (DE); Lahn, Wolfgang, Dipl.-Ing., 47877 Willich (DE); Schaper, Ulf-Armin, Dr. Dipl.-Chem., 47804 Krefeld (DE); Scheffler, Karl-Heinz, 40589 Düsseldorf (DE); Wilsch-Irrgang, Anneliese, Dr. Dipl.-Chem., 40593 Düsseldorf (DE)

(56) Entgegenhaltungen:
- EP-A- 0 507 047
- EP-A- 0 516 508
- DE-A- 4 417 476
- DATABASE WPI Week 9442 Derwent Publications Ltd., London, GB; AN 94-337523 XP002082856 "Transparent microemulsion for cleaning agents and cosmetics, etc. - comprises sucrose fatty acid ester, alkyl glucoside surfactant, oil component and water" & JP 06 262060 A (MITSUBISHI) , 20. September 1994
- DATABASE WPI Week 8926 Derwent Publications Ltd., London, GB; AN 89-189509 XP002082857 "Methyl 4,6-O-3-(1-methylcyclohexen-4yl) butylidene)-D-glucoside useful as perfume fixer and non-toxic to skin" & JP 01 128992 A (KANEBO), 22. Mai 1989

## Beschreibung

Die Erfindung betrifft Parfümölkonzentrate in Form von transparenten bis klarflüssigen Mikroemulsionen, deren Tröpfchendurchmesser im wesentlichen unter 100 nm liegt und die für Licht einer Wellenlänge von 650 nm eine Transmissivität von wenigstens 90 % aufweisen. Weiter betrifft die Erfindung die Verwendung solcher Parfümölkonzentrate zur Parfümierung wäßriger Zubereitungen sowie wäßrige Zubereitungen, die durch ihren Gehalt an Parfümölkonzentraten eine verstärkte Duftübertragung auf Textilien ermöglichen.

Aus der deutschen Offenlegungsschrift **DE 41 10 506 A1** war bekannt, daß Alkylpolyglycoside zur Herstellung feinteiliger Emulsionen von etherischen Ölen geeignet sind. Die Druckschrift offenbart kein Verfahren zur Herstellung von Mikroemulsionen oder zur Emulgierung von Duftstoffen.

In der deutschen Offenlegungsschrift **DE 44 11 557 A1** ist ein Verfahren zur Herstellung von Mikroemulsionen beschrieben, bei dem als Ölkörper ein Dialkylether verwendet wird. In der deutschen Offenlegungsschrift **DE 44 17 476 A1** sind Mikroemulsionen beschrieben, deren Ölkomponente ganz oder überwiegend aus Kohlenwasserstoffen besteht und die als Emulgatoren ein Alkylglycosid und einen Fettsäure-Polyol-Partialester enthalten.

Parfümöle sind nur wenig wasserlöslich, so daß für ihre klare Einarbeitung in wäßrigen Zubereitungen entweder sogenannte Solubilisatoren oder Lösungsmittel, z.B. niedere Alkohole, eingesetzt werden. Eine weitere Möglichkeit wäre die Emulgierung, die aber nur im Falle der Mikroemulsion eine klare Einarbeitung in wäßrige Systeme ermöglicht.

Bisher hat sich die Emulgierung von Duftstoffen als schwierig erwiesen, da die Duftstoffe durch ihre hohe Flüchtigkeit und die teilweise starke Polarität bei der Emulgierung Probleme bereiten. Sie erfordern entweder sehr hohe Mengen an Emulgatoren oder die Mitverwendung wasserlöslicher organischer Lösungsmittel.

Es wurde nun überraschenderweise festgestellt, daß sich Parfümöle in die Form transparenter bis klarflüssiger Mikroemulsionen überführen lassen, deren Tröpfchengröße im wesentlichen zwischen 10 und 100 nm liegt, wenn man das Parfümöl zusammen mit einer speziellen Co-Ölkomponente unter Verwendung von Alkylglycosiden emulgiert.

Gegenstand der Erfindung sind Parfümölkonzentrate in Form transparenter wäßriger Mikroemulsionen, deren Tröpfchengröße im wesentlichen zwischen 10 und 100 nm liegt und die einen Gehalt von
(A) 10 - 50 Gew.-% eines Parfümöls
(B) 1 - 10 Gew.-% einer Co-Ölkomponente
(C) 1 - 30 Gew.-% eines Emulgators vom Typ der Alkylglycoside der Formel
   RO - (G)ₓ, in der R eine C₈-C₂₂-Alkylgruppe und (G)ₓ einen Oligosaccharidrest mit einem mittleren Oligomerisationsgrad, x von 1 bis 2 ist, sowie gegebenenfalls
(D) bis zu 10 Gew.-% eines nichtionischen oder kationischen Co-Emulgators,
wobei das Gewichtsverhältnis von Parfümöl zu Co-Ölkomponente A : B = 10 : 1 bis 2 : 1 ist, aufweisen.

Mikroemulsionen sind optisch isotrope, thermodynamisch stabile Systeme, die wasserunlösliche Öle, Emulgatoren und Wasser enthalten. Das klare bzw. transparente Aussehen der Mikroemulsionen ist eine Folge der geringen Tröpfchengröße der dispergierten Öle, die im wesentlichen unter 300 nm liegt, wobei im Bereich zwischen 100 und 300 nm feinteilige, in der Durchsicht braunrot und im Auflicht bläulich schimmernde Mikroemulsionen und in dem hier besonders bevorzugten Bereich von 10 - 100 nm weitgehend optisch klare Mikroemulsionen vorliegen. Der optische Eindruck der klaren Transparenz ist dann besonders gut, wenn die Transmissivität der Emulsion für Licht der Wellenlänge von 650 nm bei mindestens 90 % liegt.

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 - 18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Als Co-Ölkomponente eignen sich prinzipiell alle für die Kosmetik geeigneten, natürlichen und synthetischen Ölkomponenten, z.B. vom Typ der Paraffinöle, der Pflanzenöle (Triglyceridöle), der flüssigen Wachse, Jojobaöl, der synthetische Fettsäure- und Fettalkoholester, der Dicarbonsäureester, der Ester von Diolen und Polyolen, der linearen und verzweigten Fettalkohole und der Dialkylether. Es stellte sich aber heraus, daß sich Mikroemulsionen besonders leicht bilden, wenn als Co-Ölkomponente ein Dialkylether mit insgesamt 12 - 24 C-Atomen in einer Menge von wenigstens 0,5 Gew.-% enthalten ist. Noch besser eignet sich als Co-Ölkomponente ein Gemisch aus einem Dialkylether mit insgesamt 12 - 24 C-Atomen und einem einwertigen, primären Alkohol mit 12-36 C-Atomen.

Als Dialkylether eignen sich insbesondere solche mit linearen primären Alkylgruppen mit je 6 - 12 C-Atomen, insbesondere die symmetrischen Di-n-Alkylether, wie z.B. Di-n-Octylether. Als einwertige primäre Alkohole eignen sich bevorzugt flüssige, einfach verzweigte Alkohole wie z.B. 2-Hexyl-decanol oder 2-Octyl-dodecanol.

Dialkylether und Alkanol werden bevorzugt in einem Gewichtsverhältnis von 9 : 1 bis 7 : 3 als Co-Ölkomponente verwendet. Das Gewichtsverhältnis von Ölkomponente zu Co-Ölkomponente ist bevorzugt von 3 : 1 bis 2 : 1.

Als nichtionogene Emulgatoren zur Herstellung der erfindungsgemäßen Mikroemulsionen werden Alkyloligoglycoside verwendet. Alkyloligoglycoside, ihre Herstellung und Verwendung als oberflächenaktive Stoffe sind beispielsweise aus **DE 19 43 689 A1** oder aus **DE 38 27 543 A1** bekannt.

Bezüglich des Glycosidrestes gilt, daß sowohl Monoglycoside, bei denen ein Zuckerrest glycosidisch an den Fettalkohol gebunden ist, als auch oligomere Glycoside mit einem mittleren Oligomerisationsgrad bis etwa 2 geeignet sind. Geeignete Alkyloligoglycoside sind solche der Formel RO - (G)ₓ, worin RO den aliphatischen, linearen Rest eines primären Fettalkohols mit 8 - 22, bevorzugt mit 10 - 16 C-Atomen, und (G)ₓ einen Oligoglycosid-Rest mit einem mittleren Oligomerisationsgrad x von 1 bis 2 ist. Als Glycosid-Rest ist in den handelsüblichen Alkyloligoglycosiden der Glucosidrest enthalten. Das Gewichtsverhältnis von Parfümöl (A) zu Alkylglycosid (C) liegt bevorzugt im Bereich von 0,5 : 1 bis 2 : 1.

Obwohl die Herstellung der erfindungsgemäßen Parfümölkonzentrate bei Verwendung des Dialkylethers als Co-Ölkomponente auch ohne weitere Co-Emulgatoren gelingt, kann es bei Verwendung anderer Co-Ölkomponenten erforderlich sein, einen Co-Emulgator einzusetzen. Das Gewichtsverhältnis von Alkylglycosiden zu Co-Emulgatoren sollte jedoch bevorzugt größer als 5 : 1 sein. Als Co-Emulgatoren eignen sich lipophile, nichtionogene Emulgatoren oder kationische Emulgatoren.

Als lipophile nichtionische Co-Emulgatoren können z.B. Fettsäure-Polyol-Partialester von Fettsäuren mit 10 - 18 C-Atomen und einem Polyol mit 2 - 6 C-Atomen und 2 - 6 Hydroxylgruppen verwendet werden. Beispiele für solche Co-Emulgatoren sind z.B. Sorbitan-monolaurat, Glycerin-monolaurat, Methylglucosid-monomyristat, Propylenglycol-monopalmitat sowie die Anlagerungsprodukte von 1 - 4 Mol Ethylenoxid an solche Polyol-Partialester.

Besonderes anwendungstechnisches Interesse kommt solchen erfindungsgemäßen Parfümölkonzentraten zu, die als Co-Emulgatoren kationische Emulgatoren vom Typ der quartären Ammoniumsalze enthalten. Solche kationischen Co-Emulgatoren verleihen den winzigen Mikroemulsions-Tröpfchen nämlich eine positive Ladung und bewirken auf diese Weise eine verstärkte Adsorption solcher Parfümöle aus der Emulsionsphase an negativ geladene Oberflächen, z.B. von Textilfasern.

Geeignete kationische Co-Emulgatoren sind z.B. quartäre Ammoniumtenside der allgemeinen Formel II 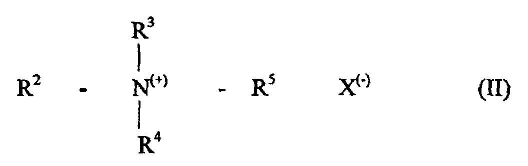 in der R² eine Alkylgruppe mit 12 - 22 C-Atomen oder eine Gruppe der Formel 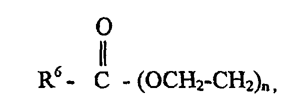 R³ und R⁴ unabhängig voneinander Alkylgruppen mit 1 - 4 C-Atomen, Hydroxyalkylgruppen mit 2 oder 3 C-Atomen oder eine Gruppe R² sind und R⁵ eine Alkylgruppe mit 1 - 4 C-Atomen oder eine Hydroxylalkylgruppe mit 2 oder 3 C-Atomen oder eine Benzylgruppe ist und X⁽⁻⁾ ein Chlorid-, Bromid- oder Methoxysulfation ist und R⁶CO eine C₁₂-C₂₂-Fettacylgruppe und n = 1 bis 4 ist.

Unter den Verbindungen, die der allgemeinen Formel II zugerechnet werden können, kommt den handelsüblichen Produkten wie z.B. dem Cetyl-trimethylammoniumchlorid, dem Benzalkoniumchlorid, dem Distearyl-dimethylammoniumchlorid und insbesondere den biologisch leicht abbaubaren sogenannten Esterquats die größte Bedeutung als Co-Emulgatoren zu. Als Esterquats werden die quaternierten Veresterungsprodukte des Diethanolamins, des Triethanolamins mit Fettsäuren verstanden, die z.B. unter Warenzeichen wie Stepantex®VS90 oder Dehyquart®AU46 im Handel sind.

Außer den genannten quartären Ammoniumsalzen können aber auch andere bekannte kationische Tenside und Emulgatoren als Co-Emulgatoren im Sinne der Erfindung eingesetzt werden.

In den erfindungsgemäßen Parfümölkonzentraten ist das Parfümöl bevorzugt in einer Menge von 15 - 30 Gew.-%, bezogen auf die gesamte Mikroemulsion, enthalten.

Auf die Mitwirkung niederer Alkohole kann dabei sehr weitgehend verzichtet werden. Die erfindungsgemäßen Konzentrate sind daher von niederen Alkoholen frei. Soweit kleinere Mengen z.B. durch die Parfümöle selbst oder durch andere Rohstoffe, z.B. durch die kationischen Co-Emulgatoren, in die Konzentrate gelangen, sollte der Anteil solcher Alkohole mit 1 - 4 C-Atomen im Konzentrat weniger als 2 Gew.-% betragen.

Die Herstellung der erfindungsgemäßen Parfümölkonzentrate erfolgt in einfacher Weise dadurch, daß man die wasserlöslichen Komponenten in Wasser, die öllölichen Komponenten gegebenenfalls bei erhöhter Temperatur mit dem Parfümöl in Lösung bringt und die beiden Lösungen bei einer möglichst niedrigen Temperatur intensiv mischt. Wenn man Co-Emulgatoren verwendet, die bei 10°C - 30°C flüssig sind, so kann die Verarbeitung bevorzugt bei Raumtemperatur von 20°C - 25°C erfolgen. Bei Mitverwendung von z.B. Glycerinmonopalmitat, einem Produkt von wachsartiger Konsistenz, als Co-Emulgator, bringt man diesen bei etwa 65°C mit dem Parfümöl in Lösung und emulgiert dann auch bei dieser Temperatur mit der wäßrigen Phase. Nach dem Abkühlen erhält man das erfindungsgemäße Parfümölkonzentrat in Form einer verhältnismäßig dünnflüssigen Mikroemulsion, in die weitere wasserlösliche Hilfsmittel eingerührt werden können.

Solche wasserlöslichen Hilfsmittel können z.B. Konservierungsstoffe, wasserlösliche Verdickungsmittel, z.B. Hydrocolloide wie wasserlösiche Gumme, Cellulosederivate oder wasserlösliche synthetische Polymere sein. Auch wasserlösliche Puffersalze oder Elektrolyte können nachträglich eingerührt werden. Eine weitere Gruppe von Hilfsmitteln sind die wasserlöslichen Polyole mit 2 - 8 C-Atomen und 2 - 6 Hydroxylgruppen. Solche geeigneten Polyole sind z.B. Ethylenglycol, 1,2-Propylenglycol, Glycerin, Erythrit, Sorbit, Methylglucosid, Diglycerin, Trimethylpropan und Pentaerythrit. Auch Polyethylenglycole oder Anlagerungsprodukte von Ethylenoxid an die genannten Polyole können als Hilfsmittel enthalten sein.

Öllösliche Hilfsmittel, z.B. antimikrobielle, deodorierende, hautkosmetische oder dermatologische Wirkstoffe sollten der Ölphase vor der Emulgierung zugesetzt werden.

Die erfindungsgemäßen Parfümöl-Konzentrate lassen sich sowohl mit Wasser verdünnen als auch wäßrigen Zubereitungen zusetzen, ohne daß es zur Koaleszenz der emulgierten Parfümöle kommt. Ein anwendungstechnisch besonders bedeutender Aspekt der Erfindung ist daher die Verwendung der erfindungsgemäßen Parfümölkonzentrate zur Beduftung wäßriger Zubereitungen. Solche Zubereitungen können z.B. Flüssigwaschmittel oder kosmetische Reinigungsmittel, wie Schaum- und Duschbadformulierungen, flüssige Seifen, Shampoos oder andere wäßrige Körperreinigungsmittel sein.

Ein weiteres Anwendungsgebiet, insbesondere für erfindungsgemäße Parfümölkonzentrate mit kationischen Co-Emulgatoren, ist die Beduftung von wäßrigen Textilnachbehandlungsmitteln, z.B. von Wäscheweichspülmitteln. Diese wäßrigen Textilnachbehandlungsmittel zeichnen sich durch ihren Gehalt an den erfindungsgemäßen Parfümölmikroemulsionen gegenüber herkömmlichen Nachbehandlungsmitteln dadurch aus, daß sie eine deutlich verstärkte Duftübertragung auf die behandelten Textilien und einen länger anhaltenden Duft der Textilien ermöglichen. Wäßrige Wäschenachbehandlungsmittel, insbesondere Weichspülerkonzentrate, enthalten die erfindungsgemäßen Parfümöl-Mikroemulsionen in Mengen von mehr als 1 Gew.-%, bezogen auf das gesamte Mittel, vorzugsweise sind die erfindungsgemäßen Parfümöl-Mikroemulsionen in Mengen von mehr als 2 Gew.-% enthalten. Die Einsatzmenge der erfindungsgemäßen Parfümöl-Mikroemulsionen richtet sich einerseits nach ihrem Parfümgehalt, andererseits nach dem gewünschten Parfümgehalt in den zu beduftenden wäßrigen Zubereitungen. Je nach ihrer Menge an Aktivsubstanz unterscheidet man bei Textilnachbehandlungsmitteln zwischen "Normalware" und "Konzentraten". Bei Aktivsubstanzgehalten oberhalb von 6 Gew.-%, bezogen auf die fertige Formulierung, liegt ein Konzentrat vor, während unterhalb dieser Grenze von "Normalware" gesprochen wird. In Wäscheweichspülmitteln kommen als Aktivsubstanzen sämtliche kationischen und/oder nichtionischen Softener in Frage. Hierzu zählen insbesondere quartäre Ammoniumverbindungen (QAV's) wie beispielsweise Distearyldimethylammoniumchlorid, Fettasäureaminoamide (Talgamid® B, Henkel), Glycerylstearate (Edenor® GMS, Cutina® GMS, Henkel), die sogenannten "Esterquats", beispielsweise Methyldiethanolamin-QAV's, Dimethylpropandiol-QAV's sowie weiterhin die textilweichmachenden Tone.

Für Weichspüler sind die Parfümöl-Mikroemulsionen vorzugsweise in den Mengen einzusetzen, daß die fertigen Mittel Parfümgehalte zwischen 0,05 bis 1 Gew.-%, vorzugsweise von 0,1 bis 0,5 Gew.-%, bezogen auf den gesamten Weichspüler, aufweisen. Bei Weichspülerkonzentraten sind die Mengen so zu wählen, daß die Menge an Parfüm, bezogen auf das Weichspülerkonzentrat, zwischen 0,2 und 2,5 Gew.-%, vorzugsweise zwischen 0,4 und 1,5 Gew.-%, beträgt. Bei den Parfümöl-Mikroemulsionen, die 20 Gew.-% ihres Gewichts an Parfüm enthalten, sind also Einsatzbereiche der Parfümöl-Mikroemeulsionen zwischen 0,25 und 5 Gew.-% (Für Weichspüler, bezogen auf das Gewicht des fertig konfektionierten Weichspülers) bzw. zwischen 1 und 12,5 Gew.-% (Für Weichspülerkonzentrate, bezogen auf das Gewicht des fertig konfektionierten Konzentrats) möglich.

Die mit den erfindungsgemäßen Parfümöl-Mikroemulsionen zu beduftenden Weichspüler oder Weichspülerkonzentrate können dabei sämtliche üblichen Inhaltsstoffe solcher Avivagemittel enthalten. Unverträglichkeiten der erfindungsgemäßen Parfümöl-Mikroemulsionen mit üblichen Inhaltsstoffen von Wasch- und Reinigungsmitteln existieren nicht. Auch können sämtliche üblicherweise in Avivagemitteln eingesetzten Mittel eingesetzt werden, die den Textilien den gewünschten "weichen Griff" verleihen. Neben den Alkyl-N(2-hydroxyethyl)-N,N-di(acyloxyethyl)ammonium-Salzen (sogenannte "Esterquats") ist selbstverständlich auch ein Einsatz der bekannten quartären Ammoniumverbindungen, der Amine, Aminoamide, Glycerylstearate und der Tone, die eine Avivagewirkung aufweisen, möglich. Gängige Textilweichspüler, die mit den erfindungsgemäßen Mikroemulsionen beduftet werden können, sind beispielsweise in den Patentanmeldungen EP-A-0 409 504 ("Esterquat", Unilever), WO94/20597 ("Esterquat", Procter & Gamble), EP-A-0 240 727 ("Esterquat"/Fettsäure(seife), Henkel), EP-A-0 394 133 ("Esterquat"/QAV/Fettalkohol, Colgate), EP-A-0 380 406 (Zuckerester, Colgate), EP-A-0 407 040 (langkettige QAV/Fettalkohol, Colgate), EP-A-0 584 692 (Fettsäure/Fettalkohol/QAV, Mäurer + Wirtz) sowie EP-A-0 707 059 (alkoxyliertes Glycerin/"Esterquat"/Fettsäure, Kao Corp.) beschrieben.

Aber auch die Parfümierung von Textilbehandlungsflotten selbst kann mit Hilfe der erfindungsgemäßen Parfümölkonzentrate erfolgen. So können die Parfümöl-Mikroemulsionen auch ohne zusätzliche Wirk- und Hilfsstoffe im letzten Spülbad eingesetzt werden, um Textilien zu beduften, ohne dabei Weichspüleffekte zu erzielen. Schießlich kann man die Parfümölkonzentrate für alle Aufgaben der Beduftung technischer und kosmetischer Produkte verwenden, insbesondere dort, wo man keine alkoholischen Zubereitungen einsetzen kann.

Die erfindungsgemäßen Mikroemulsionen lassen sich auch über herkömmliche Trockner auf Textilien oder andere Substrate aufbringen. Hierbei können die Mikroemulsionen oder weiter verdünnte Varianten beispielsweise als Spray oder über einen Zerstäuber eingebracht werden. Außerdem besteht die Möglichkeit, die erfindungsgemäßen Mikroemulsionen auf einen Träger aufzubringen und so Granulate oder "imprägnierte" Tücher bereitzustellen, die mit oder ohne Zusatz von Avivagemitteln zur Textilbehandlung in Trocknern eingesetzt werden.

Die folgenden Beispiele sollen den Gegenstand der Erfindung näher erläutern:

### Beispiele

Es wurden die folgenden Parfümöle verwendet:

| **Parfümöl I** | |
|---|---|
| Bergamotteöl | 50 Gew.-% |
| Dihydromyrcenol | 50 Gew.-% |
| | 100 Gew.-% |

| **Parfümöl II (Maiglöckchen-Komposition)** | |
|---|---|
| Lilial | 15,0 Gew.-% |
| Lyral | 20,0 Gew.-% |
| Citronellol | 10,0 Gew.-% |
| Phenylethylalkohol | 10,0 Gew.-% |
| ∝-Hexylzimtaldehyd | 10,0 Gew.-% |
| Geraniol | 5,0 Gew.-% |
| Benzylaceton | 3,0 Gew.-% |
| Cyclamenaldehyd | 2,0 Gew.-% |
| Linalool | 2,0 Gew.-% |
| Boisambrene Forte | 1,7 Gew.-% |
| Ambroxan | 0,2 Gew.-% |
| Indol | 0,1 Gew.-% |
| Hedione | 16,0 Gew.-% |
| Sandelice | 5,0 Gew.-% |
| | 100 Gew.-% |

| **Parfümöl III (Typ Drakkar Noir)** | |
|---|---|
| Bergamotteöl | 15,0 Gew.-% |
| Dihydromyrcenol | 20,0 Gew.-% |
| Citronenöl Messina | 7,5 Gew.-% |
| Mandarinenöl | 2,5 Gew.-% |
| Orangenöl süß | 5,0 Gew.-% |
| Allylamylglycolat | 2,0 Gew.-% |
| Cyclovertal | 0,5 Gew.-% |
| Lavandinöl grosso | 2,5 Gew.-% |
| Muskateller Salbeiöl | 1,0 Gew.-% |
| Lilial | 2,0 Gew.-% |
| β-Damascone | 0,1 Gew.-% |
| Geraniumöl Bourbon | 3,0 Gew.-% |
| Hedione | 5,0 Gew.-% |
| Cyclohexylsalicylat | 4,0 Gew.-% |
| Vertofix Coeur | 10,0 Gew.-% |
| Iso-E-Super | 5,0 Gew.-% |
| Ambroxan | 1,6 Gew.-% |
| Fixolide NP | 10,0 Gew.-% |
| Evernyl | 1,0 Gew.-% |
| Dipropylenglycol | 2,3 Gew.-% |
| | 100,0 Gew.-% |

| **Parfümöl IV (blumig-fruchtige Rosen-Komposition)** | |
|---|---|
| Phenylethylalkohol | 52,0 Gew.-% |
| DMBCA | 2,5 Gew.-% |
| Iraldein gamma | 5,0 Gew.-% |
| Phenylessigsäure | 0,5 Gew.-% |
| Geranylacetat | 2,0 Gew.-% |
| Benzylacetat | 3,0 Gew.-% |
| Rosenoxid L 10 % in DPG | 2,5 Gew.-% |
| Romilat | 2,0 Gew.-% |
| Irotyl | 0,5 Gew.-% |
| Cyclohexylsalicylat | 20,0 Gew.-% |
| Floramat | 10,0 Gew.-% |
| | 100,0 Gew.-% |

| **Parfümöl V** | |
|---|---|
| Naphthylethylether | 0,4 Gew.-% |
| Tricyclodecenylacetat | 4,4 Gew.-% |
| Tricyclodecenylpropionat | 2,2 Gew.-% |
| Floramat | 5,5 Gew.-% |
| Amylzimtaldehyd (Alpha) | 14,3 Gew.-% |
| Cyclohexylsalicylat | 25,3 Gew.-% |
| Vertofix Coeur | 11,0 Gew.-% |
| Boisambrene Forte | 4,0 Gew.-% |
| Fixolide® (Fa. Givaudan) | 32,9 Gew.-% |
| | 100,0 Gew.-% |

### Beispiele für Parfümöl-Mikroemulsionskonzentrate

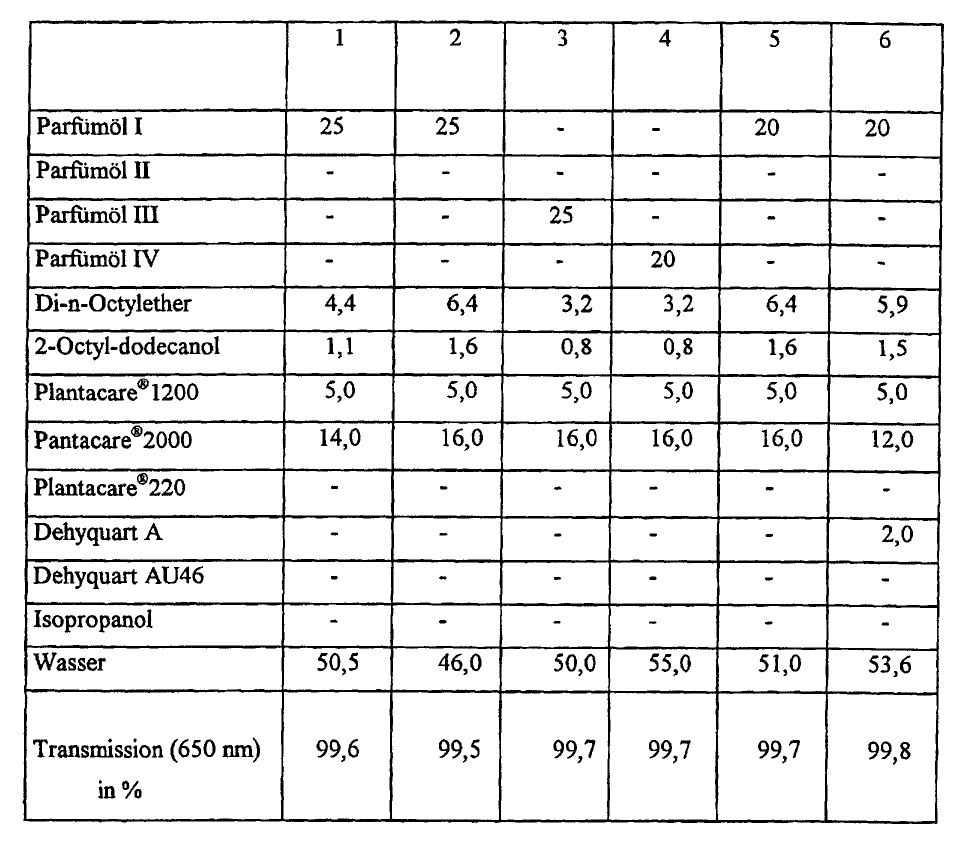

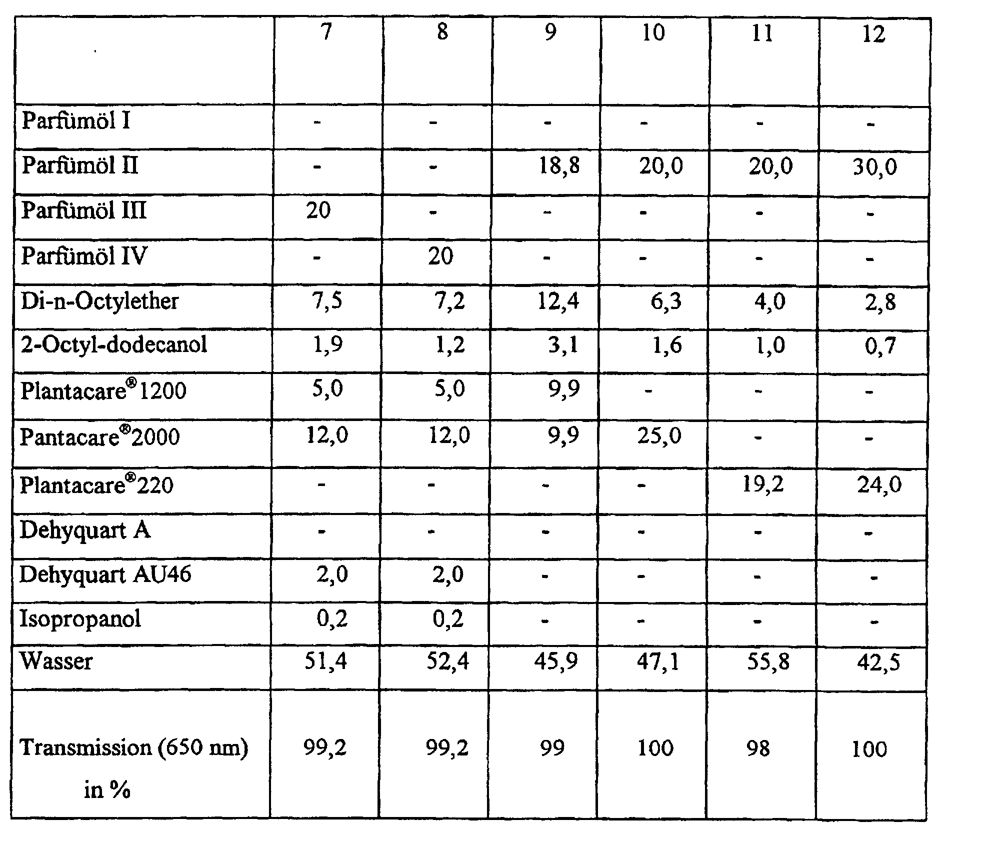

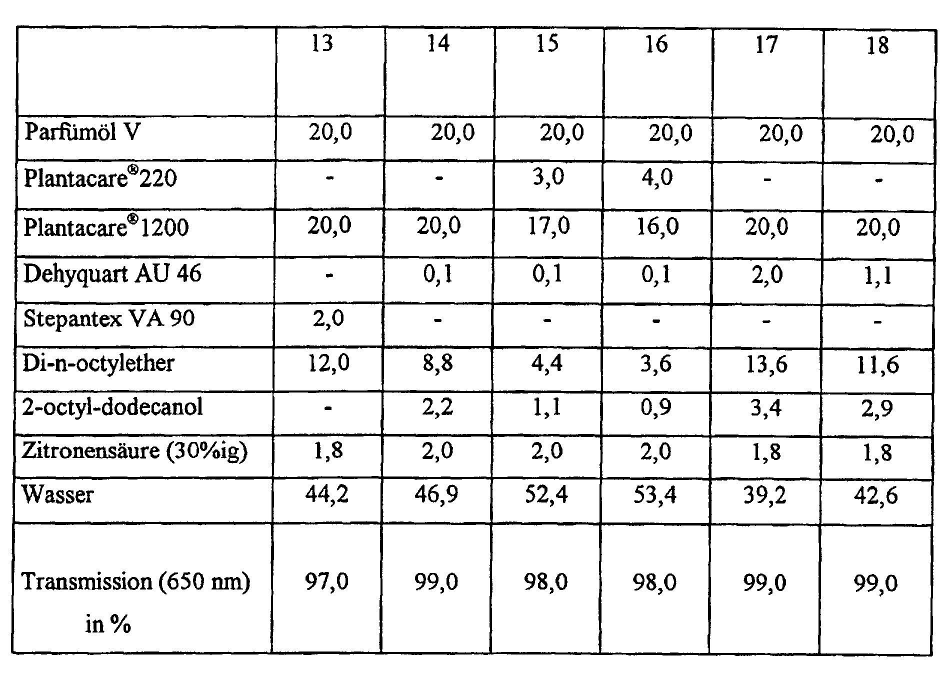

Die Angaben beziehen sich auf Gew.-% Aktivsubstanz (auch bei den angegebenen wäßrigen Handelsprodukten).

Es wurden folgende Handelsprodukte verwendet:

| | |
|---|---|
| Plantacare®2000 | C₈-C₁₆-Alkyl-oligo-(1.4)-glucosid, 50 %-ig in Wasser |
| Plantacare®1200 | C₁₂-C₁₆-Alkyl-oligo-(1.4)-glucosid, 50 %-ig in Wasser |
| Plantacare®220 | C₈-C₁₀-Alkyl-oligo-(1.5)-glucosid, 63 %-ig in Wasser |
| Stepantex VA 90 | Methyl-N(2-hydroxyethyl)-N,N-di(talgacyloxyethyl)ammoniummethosulfat, 90%-ig in Isopropanol |
| Dehyquart A | Cetyl-trimethylammonium-chlorid, 25 %-ig in Wasser |
| Dehyquart AU46 | Dipalmitoleyloxyethyl-hydroxyethyl-methylammonium-methoxysulfat, 90 %-ig in Isopropanol. |

Es wurden je zwei wäßrige Wäschenachbehandlungsmittel (Weichspüler) hergestellt, die einmal als Duftkomponente die erfindungsgemäßen Parfümöl-Mikroemulsionen (E1 und E2) und zum Vergleich normales Parfümöl (V1 und V2) enthielten. Hierbei wurden die Mengen an Parfüm und erfindungsgemäßer Parfümöl-Mikroemulsion so gewählt, daß jeweils die gleiche Menge Duftstoff in den Weichspüler-Zusammensetzungen enthalten war. Die Beduftung von behandelten Textilien (Baumwolle) wurde als subjektiver Geruchseindruck von Parfümeuren und durch das OEB-Panel beurteilt. Hierbei geben die Zahlenwerte in der Bewertungstabelle die Anzahl der Parfümeure an, die die mit dem jeweiligen Mittel behandelten Textilien als "stärker duftend" eingestuft haben.Da in den unterschiedlichen Riechtests eine verschiedene Zahl von Parfümeuren anwesend war, addieren sich die Werte in den Spalten "Parfümeure" nicht immer auf den gleichen Wert. die erste Spalte (1 Tag) ist folglich so zu lesen, daß von 7 Parfümeuren 6 die mit dem Mittel E1 behandelten Textilien als stärker duftend bewertet haben. Beim OEB-Test ("Olfactory Evaluation Board") wurden die behandelten Textilien von 27 Testpersonen (keine Parfümeure) bewertet. Der Zahlenwert in der Tabelle gibt die Anzahl der Personen wieder, die die mit dem jewiligen Mittel behandelten Textilien als "stärker duftend" eingestuft haben.

### Wäschenachbehandlungsmittel (Weichspülerkonzentrat) - Zusammensetzung:

| | **E1** | **V1** | **E2** | **V2** |
|---|---|---|---|---|
| Stepantex VA 90 | 4,50 | 4,50 | 16,20 | 16,20 |
| MgCl₂ x 6 H₂O | - | - | 0,30 | 0,30 |
| Farbstoff | 0,003 | 0,003 | 0,01 | 0,01 |
| Parfüm-Zubereitung | - | 0,35 | - | 0,75 |
| Parfümöl-Mikroemulsion | 1,75 | - | 3,75 | - |
| Wasser | 93,747 | 91,747 | 83,14 | 82,74 |

Die Angaben beziehen sich auf Gew.-% Aktivsubstanz

### Duftverstärkung auf trockener Wäsche

| | **Präferenz der Intensität** | | | |
|---|---|---|---|---|
| **Weichspülkonzentrat** | **Parfümeure** | | | **OEB** |
| | 1 Tag | 3 Tage | 7 Tage | 3 Tage |
| **E1** | 6 | 5 | 4 | 26 |
| **V1** | 1 | 0 | 2 | 1 |
| | | | | |
| **E2** | 6 | 4 | 5 | 20 |
| **V2** | 1 | 2 | 1 | 7 |

## Patentansprüche

1. Parfümölkonzentrate in Form transparenter wäßriger Mikroemulsionen, deren Tröpfchengröße im wesentlichen zwischen 10 und 100 nm liegt, **gekennzeichnet durch** einen Gehalt von
(A) 10 - 50 Gew.-% eines Parfümöls
(B) 1 - 10 Gew.-% einer Co-Ölkomponente
(C) 1 - 30 Gew.-% eines Emulgators vom Typ der Alkylglycoside der Formel
RO - (G)ₓ, in der R eine C₈-C₂₂-Alkylgruppe und (G)ₓ eine Oligosaccharidgruppe mit einem mittleren Oligomerisationsgrad,
x von 1 bis 2 darstellt, sowie gegebenenfalls
(D) bis zu 10 Gew.-% eines nichtionischen oder kationischen Co-Emulgators,
wobei das Gewichtsverhältnis von Parfümöl zu Co-Ölkomponente A : B = 10 : 1 bis 2 : 1 ist.

2. Parfümkonzentrat gemäß Anspruch 1, **dadurch gekennzeichnet, daß** als Co-Ölkomponente ein Dialkylether mit insgesamt 12 - 24 C-Atomen in einer Menge von wenigstens 0,5 Gew.-% enthalten ist.

3. Parfümölkonzentrat nach einem der Ansprüche 1 oder 2, **dadurch gekennzeichnet, daß** als Co-Ölkomponente ein Gemisch aus einem Dialkylether mit insgesamt 12 - 24 C-Atomen und einem einwertigen primären Alkohol mit 12 - 36 C-Atomen enthalten ist.

4. Parfümölkonzentrat nach einem der Ansprüche 1 bis 3, **dadurch gekennzeichnet, daß** das Parfümöl in einer Menge von 15 - 30 Gew.-% enthalten ist.

5. Parfümölkonzentrat nach einem der Ansprüche 1 bis 4, **dadurch gekennzeichnet, daß** es von niederen Alkoholen weitgehend frei ist oder weniger als 2 Gew.-% niederer Alkohole mit 1 - 4 C-Atomen enthält.

6. Parfümölkonzentrat nach einem der Ansprüche 1 bis 5, **dadurch gekennzeichnet, daß** das Gewichtsverhältnis von Alkylglycosiden zu Co-Emulgatoren größer als 5 : 1 ist.

7. Parfümölkonzentrat nach einem der Ansprüche 1 bis 6, **dadurch gekennzeichnet, daß** das Gewichtsverhältnis von Parfümöl zu Alkylglycosid im Bereich von 0,5 : 1 bis 2 : 1 liegt.

8. Verwendung der Parfümölkonzentrate gemäß Anspruch 1 bis 7 zur Parfümierung wäßriger Zubereitungen.

9. Wäßriges Textilnachbehandlungsmittel, enthaltend mindestens 1 Gew.-%, vorzugsweise mindestens 2 Gew.-% eines Parfümölkonzentrates nach einem der Ansprüche 1 bis 7.

10. Wäßriger Weichspüler, enthaltend ein Parfümölkonzentrat nach einem der Ansprüche 1 bis 7, **dadurch gekennzeichnet, daß** die Menge an Parfüm bezogen auf den Weichspüler 0,05 bis 1 Gew.-%, vorzugsweise 0,1 bis 0,5 Gew.-% beträgt.

11. Wäßriges Weichspülerkonzentrat, enthaltend ein Parfümölkonzentrat nach einem der Ansprüche 1 bis 7, **dadurch gekennzeichnet, daß** die Menge an Parfüm bezogen auf das Weichspülerkonzentrat 0,2 bis 2,5 Gew.-%, vorzugsweise 0,4 bis 1,5 Gew.-% beträgt.

## Claims

1. Perfume oil concentrates in the form of transparent aqueous microemulsions with a droplet size of largely between 10 and 100 nm, **characterized by** a content of
(A) 10 to 50% by weight of a perfume oil,
(B) 1 to 10% by weight of a co-oil component,
(C) 1 to 30% by weight of an emulsifier of the alkyl glycoside type corresponding to the formula RO - (G)ₓ, in which R is a C₈₋₂₂ alkyl group and (G)ₓ is an oligosaccharide group with a mean degree of oligomerization x of 1 to 2, and optionally
(D) up to 10% by weight of a nonionic or cationic co-emulsifier,
the ratio by weight of perfume oil to co-oil component A:B being 10:1 to 2:1.

2. A perfume oil concentrate as claimed in claim 1, **characterized in that** a dialkyl ether containing a total of 12 to 24 carbon atoms is present in a quantity of at least 0.5% by weight as the co-oil component.

3. A perfume oil concentrate as claimed in claim 1 or 2, **characterized in that** a mixture of a dialkyl ether containing a total of 12 to 24 carbon atoms and a monohydric primary alcohol containing 12 to 36 carbon atoms is present as the co-oil component.

4. A perfume oil concentrate as claimed in any of claims 1 to 3, **characterized in that** the perfume oil is present in a quantity of 15 to 30% by weight.

5. A perfume oil concentrate as claimed in any of claims 1 to 4, **characterized in that** it is substantially free from lower alcohols or contains less than 2% by weight of lower C₁₋₄ alcohols.

6. A perfume oil concentrate as claimed in any of claims 1 to 5, **characterized in that** the ratio by weight of alkyl glycosides to co-emulsifiers is greater than 5:1.

7. A perfume oil concentrate as claimed in any of claims 1 to 6, **characterized in that** the ratio by weight of perfume oil to alkyl glycoside is in the range from 0.5:1 to 2:1.

8. The use of the perfume oil concentrates claimed in claims 1 to 7 for perfuming water-containing preparations.

9. A water-containing laundry aftertreatment preparation containing at least 1% by weight and preferably at least 2% by weight of the perfume oil concentrate claimed in any of claims 1 to 7.

10. A water-containing fabric softener containing the perfume oil concentrate claimed in any of claims 1 to 7, **characterized in that** the quantity of perfume, based on fabric softener, is in the range from 0.05 to 1% by weight and preferably in the range from 0.1 to 0.5% by weight.

11. A water-containing fabric softener concentrate containing the perfume oil concentrate claimed in any of claims 1 to 7, **characterized in that** the quantity of perfume, based on fabric softener concentrate, is in the range from 0.2 to 2.5% by weight and preferably in the range from 0.4 to 1.5% by weight.

## Revendications

1. Concentrés d'essence de parfum sous forme de microémulsions aqueuses transparentes dont la taille des gouttelettes se situe essentiellement entre 10 et 100 nm,
**caractérisés**
une teneur de :
(A) 10 à 50 % en poids d'une essence en poids
(B) 1 à 10 % en poids d'un co-composant huileux
(C) 1 à 30 % en poids d'un agent émulsionnant du type des alkyl glycosides de formule RO-(G)ₓ dans laquelle R représente un groupe alkyle en C₈-C₂₂ et (G)ₓ un groupe oligosaccharide ayant un degré d'oligomérisation moyen x de 1 à 2, ainsi qu'éventuellement,
(D) jusqu'à 10 % en poids d'agent co-émulsionnant non ionique ou cationique,
le rapport en poids de l'essence de parfum pour le co-composant huileux étant A:B = 10:1 à 2:1.

2. Concentré de parfum conformément à la revendication 1,
**caractérisé en ce que**
comme co-composant huileux un éther dialkylique ayant au total de 12 à 24 atomes de carbone est présent en une quantité d'au moins 0,5 % en poids.

3. Concentré d'essence de parfum selon l'une des revendications 1 ou 2,
**caractérisé en ce que**
comme co-composant huileux un mélange à base d'éther dialkylique ayant au total de 12 à 24 atomes de carbone et d'un alcool primaire unifonctionnel ayant de 12 à 36 atomes de carbone est présent.

4. Concentré d'essence de parfum selon d'une des revendications 1 à 3,
**caractérisé en ce que**
l'essence de parfum est présente en une quantité de 15 à 30 % en poids.

5. Concentré d'essence de parfum selon l'une des revendications 1 à 4,
**caractérisé en ce qu'**
il est largement exempt d'alcools inférieurs ou renferme moins de 2 % en poids d'alcools inférieurs ayant de 1 à 4 atomes de carbone.

6. Concentré d'essence de parfum selon l'une des revendications 1 à 5,
**Caractérisé en ce que**
le rapport pondéral des alkylglycosides au co-agent émulsionnant est plus grand que 5:1.

7. Concentré d'essence de parfum selon l'une des revendications 1 à 6,
**caractérisé en ce que**
le rapport pondéral de l'essence de parfum à l'alkylglycoside se situe dans la zone de 0,5:1 à 2:1.

8. Utilisation des concentrés d'essence de parfum conformément aux revendications 1 à 7 en vue de l'aromatisation de préparations aqueuses.

9. Produits de retraitement du textile aqueux contenant au moins 1 % en poids - de préférence au moins 2 % en poids - d'un concentré d'essence de parfum selon l'une des revendications 1 à 7.

10. Agent assouplissant aqueux contenant un concentré d'essence de parfum selon l'une des revendications 1 à 7,
**caractérisé en ce que**
la quantité en parfum - rapporté au produit de rinçage assouplissant s'élève à - 0,05 à 1 % en poids, de préférence à 0,1 à 0,5 % en poids.

11. Concentré de produit de rinçage assouplissant aqueux contenant un concentré d'essence de parfum selon l'une des revendications 1 à 7,
**caractérisé en ce que**
la quantité de parfum rapporté au concentré de produit de rinçage assouplissant s'élève à 0,2 à 2,5 % en poids - de préférence à 0,4 à 1,5 % en poids.
